# EUROPÄISCHE PATENTSCHRIFT

(11) **EP 3 405 179 B1**
(45) Veröffentlichungstag und Bekanntmachung des Hinweises auf die Patenterteilung: **09.08.2023**
(21) Anmeldenummer: 17700185.6
(22) Anmeldetag: 17.01.2017
(51) Int. Cl.: A61K 9/70, A61K 31/485, A61P 25/04

(54) **STEUERUNG DER FREISETZUNG VON WASSER AUS EINER FORMFESTEN WASSERHALTIGEN ZUSAMMENSETZUNG**
CONTROL OF THE RELEASE OF WATER FROM A SOLID COMPOSITION CONTAINING WATER
CONTRÔLE DE LA DIFFUSION D'EAU PROVENANT D'UNE COMPOSITION SOLIDE HYDRATEE

(30) Priorität: 20.01.2016 EP 16152059
(43) Veröffentlichungstag der Anmeldung: 28.11.2018
(73) Patentinhaber: LTS Lohmann Therapie-Systeme AG, 56626 Andernach (DE)
(72) Erfinder: HAMMES, Florian, 56626 Andernach (DE); EIFLER, René, 56072 Koblenz (DE)
(74) Vertreter: Schweitzer, Klaus
(86) Internationale Anmeldenummer: PCT/EP2017/050866
(87) Internationale Veröffentlichungsnummer: WO 2017/125376

(56) Entgegenhaltungen:
- DE-A1-102008 016 804
- DE-A1-102009 036 485

## Beschreibung

Die Erfindung betrifft Systeme bestehend aus einer verschlossenen flüssigkeitsdichten Verpackung vorzugsweise Folienverpackung und einer darin eingeschlossenen vorzugsweise formfesten wasserhaltigen Zusammensetzung, die nach Aktivierung eine im Wesentlichen aus Wasser bestehende flüssige Phase freisetzt, und dessen Anwendung.

Aus der WO 2009/121514 A2 sind selbstzerstörende transdermale therapeutische Systeme (TTS) bekannt, die einen Wirkstoff, ein den Wirkstoff unbrauchbar machendes wasserlösliches Agens, wie Kaliumpermanganat, und ein mechanisches Mittel zur Perforation enthalten. Das mechanische Mittel zur Perforation bewirkt, dass beim Abziehen des TTS nach Gebrauch Wasser an das den Wirkstoff unbrauchbar machende wasserlösliche Agens herantreten kann, das dann mit dem Wirkstoff in Berührung kommt und diesen dann in Anwesenheit von Wasser zerstört.

In einer Ausführungsform der Erfindung dieser WO ist unterhalb der oberen Abdeckschicht des TTS ein versiegelter Beutel mit einem Wasservorrat angeordnet, in dem sich auch das mechanische Mittel zur Perforation befinden kann. Allerdings ist die erforderliche Zugabe von Wasser bei der Herstellung eines solchen Systems aufgrund der mangelnden Dichtigkeit der Siegelrandbeutel sehr schwer zu realisieren. Aufgrund von kondensierendem Wasserdampf während des Siegelvorgangs weisen die Beutel nicht die erforderliche Dichtheit auf. Wasser ist aber zur Aktivierung des selbstzerstörenden TTS zwingend erforderlich.

Aufgabe der vorliegenden Erfindung war es, Wasser in einen Beutel zu applizieren und diesen dann dicht zu versiegeln.

Es wurde nun gefunden, dass das erforderliche sichere Einbringen von Wasser in einen Beutel und das anschließende dichte Versiegeln des Beutel überraschenderweise durch die Auswahl eines Wafers auf Basis eines niederveresterten/amidierten Pektins (NV-Perktins) gelöst werden kann (Unter einem "Wafer" versteht man hier eine film-, folien- oder oblatenartige flächenförmige vorzugsweise formfeste Zusammensetzung). Pektine mit einem Veresterungsgrad (VE°) unter 50%, d.h. mit weniger als 50% veresterten Polygalacturonsäure-Einheiten, sind in der Lage, mit Calciumionen zu gelieren. Die resultierende Gelstärke wird dabei durch Pektinmenge, Pektintyp, löslichen Trockensubstanzgehalt, pH-Wert und die Konzentration von Calciumionen bestimmt. Durch die weitere Zugabe von Calciumsalz kommt es zu einer Überschreitung des Calcium-Optimums. Dies führt zu einem spröden Gel (Bildung von Calciumpektinat, dem unlöslichen Calciumsalz des Pektins) mit starker Neigung zur Synärese (Wasseraustritt aus dem Gel). Durch die Verwendung eines solchen Wafers und durch Zugabe eines Calciumsalzes, wie Calciumchlorid tritt zeitverzögert Wasser infolge von Synärese aus der Gelstruktur des Wafers aus, so dass während des Siegelvorgangs kein Wasser verdampfen kann. Somit kann die Beuteldichtigkeit gewährgeleistet werden.

Die Erfindung betrifft daher ein System bestehend aus einer verschlossenen flüssigkeitsdichten Verpackung, vorzugsweise einer verschlossenen flüssigkeitsdichten Folienverpackung und einer darin eingeschlossenen vorzugsweise formfesten wasserhaltigen Zusammensetzung, die gegebenenfalls eine ausreichende Menge eines Aktivierungsmittels enthält, wobei die Zusammensetzung mit zeitlicher Verzögerung eine im Wesentlichen aus Wasser bestehende flüssige Phase freisetzt, vorzugsweise ein System, worin es sich bei der vorzugsweise formfesten wasserhaltigen Zusammensetzung um ein Hydrogel auf Basis eines niederveresterten Pektins oder eines niederveresterten, amidierten Pektins handelt, und wobei die Zusammensetzung eine für die zeitlich verzögerte Freisetzung der im Wesentlichen aus Wasser bestehenden flüssigen Phase ausreichende Menge eines Erdalkali- oder Erdmetallsalzes, vorzugsweise eines Calciumsalzes, insbesondere an Calciumchlorid als Aktivierungsmittel enthält.

Diese Zusammensetzungen auf Basis von Pektinen enthalten vorzugsweise 2,5 - 7,5 Gew.-% Pektin, 91 - 97 Gew.-% Wasser und 0,1 - 3 Gew.-% eines gelbildenden Erdalkali- oder Erdmetallsalzes, vorzugsweise eines Calciumsalzes, insbesondere von Calciumchlorid. Besonders bevorzugt sind 3,5 - 6,5 Gew.-% Pektin, 93 - 96 Gew.-% Wasser und 0,2 - 2 Gew.-% eines gelbildenden Erdalkali- oder Erdmetallsalzes, vorzugsweise eines Calciumsalzes, insbesondere von Calciumchlorid. Die Wasserfreisetzung wird durch Zugabe von vorzugsweise weiteren 0,1 - 3 Gew.-%, insbesondere 0,1 - 3 Gew.-% eines Erdalkali- oder Erdmetallsalzes, vorzugsweise eines Calciumsalzes, insbesondere von Calciumchlorid induziert.

Niederveresterte Pektine (NV-Pektine) sind Pektine mit weniger als 50% veresterten Polygalacturonsäure-Einheiten (VE°<50%). Sie sind in der Lage, mit Calciumionen zu gelieren. Bei amidierten Pektinen wird durch die Verwendung von Ammoniak an Stelle von Säure ein Teil der Estergruppen gegen Amidgruppen ausgetauscht. Dadurch ändern sich die Geliereigenschaften gegenüber den sauer entesterten Pektinen. NV-amidierte Pektine benötigen wie nicht-amidierte Pektine Calciumionen zur Gelierung. Sie gelieren bereits mit geringen Mengen Calciumionen ausreichend fest.

Es wurde außerdem gefunden, dass auch Hydrogele auf Basis eines depolymerisierten Guargummiprodukts, wie ^{®}Meyprogat 90, oder eines Polyacrylats, vorzugsweise Natriumpolyacrylat, wie ^{®}Aronvis-Typen, nach Zusatz von Citronensäure mittels Synärese Wasser freisetzen können. Bei der erfindungsgemäßen vorzugsweise formfesten wasserhaltigen Zusammensetzung kann es sich daher um ein Hydrogel auf Basis eines depolymerisierten Guargummiprodukts oder eines Polyacrylats handeln, bei welchem die Aktivierung durch Zusatz einer für die zeitlich verzögerte Freisetzung der im Wesentlichen aus Wasser bestehenden flüssigen Phase ausreichende Menge einer organischen Säure, vorzugsweise Citronensäure erfolgt.

Die Zusammensetzungen auf Basis von depolymerisierten Guargummiprodukten enthalten vorzugsweise 2,5 - 7,5 Gew.-% depolymerisiertes Guargummiprodukt und 91 - 97 Gew.-% Wasser. Besonders bevorzugt sind 3,5 - 6,5 Gew.-% depolymerisiertes Guargummiprodukt und 93 - 96 Gew.-% Wasser. Die Wasserfreisetzung wird durch Zugabe von 0,1 - 3 Gew.-%, insbesondere 0,1 - 3 Gew.-% einer organischen Säure, vorzugsweise Citronensäure induziert.

Die Zusammensetzungen auf Basis eines Polyacrylats, vorzugsweise Natriumpolyacrylat, wie ^{®}Aronvis-Typen enthalten vorzugsweise 10 - 34 Gew.-% Polyacrylat und 66 - 90 Gew.-% Wasser. Besonders bevorzugt sind 16 - 28 Gew.-% Polyacrylat und 72 - 84 Gew.-% Wasser. Die Wasserfreisetzung wird durch Zugabe von 0,1 - 3 Gew.-%, insbesondere 0,1 - 3 Gew.-% einer organischen Säure, vorzugsweise Citronensäure induziert.

Bei den Zusammensetzungen, bei denen die Freisetzung der im Wesentlichen aus Wasser bestehenden flüssigen Phase nach Zusatz einer ausreichenden Menge eines Aktivierungsmittels erfolgt, versteht man unter der "zeitlichen Verzögerung der Freisetzung", dass die Freisetzung nach ca. 0,5 - 6 min, vorzugsweise nach ca. 0,7 - 4 min, insbesondere nach ca. 1 - 2 min einsetzt. Durch diese Verzögerung bleibt ausreichend Zeit zum dichten Verschließen der Verpackung, vorzugsweise durch Siegeln. Der Prozess der Freisetzung ist nach ca. 1 - 3 h, vorzugsweise nach ca. 1 - 5 h, insbesondere nach ca. 1 - 10 h abgeschlossen.

Weiterhin kann es sich bei der vorzugsweise formfesten wasserhaltigen Zusammensetzung um ein gekühltes Ölsäure-Wasser-Gemisch oder ein niedrigschmelzendes Salzhydrat, vorzugsweise Calciumchlorid-Hexahydrat, Mangansulfat-Tetrahydrat, Natriumhydrogenphosphat-Dodecahydrat oder Lithiumnitrat-Trihydrat handeln. Die Aktivierung und die Wasserfreisetzung erfolgt dabei durch Erwärmen.

Die Zusammensetzungen auf Basis eines gekühlten Ölsäure-Wasser-Gemisches enthalten vorzugsweise 40 - 60 Gew.-% Ölsäure und 40 - 60 Gew.-% Wasser. Besonders bevorzugt sind 45 - 55 Gew.-% Ölsäure und 45 - 55 Gew.-% Wasser. Zu ihrer Herstellung stellt man bei Raumtemperatur eine Emulsion der Komponenten her, die an dann auf 0 - 1°C, vorzugsweise 0 - 0,5°C herunterkühlt, wodurch eine steife Masse entsteht, die bei ca. 6°C gelagert und zu Wafern weiterverarbeitet werden kann. Die Wasserfreisetzung wird durch Erwärmen auf ca. 17 - 30°C, vorzugsweise etwa auf Raumtemperatur induziert.

Die niedrigschmelzenden Salzhydrate müssen unterhalb ihres Schmelzpunktes gelagert und verarbeitet werden. So schmelzen Calciumchlorid-Hexahydrat bei ca. 30°C, Mangansulfat-Tetrahydrat bei ca. 27°C, Natriumhydrogenphosphat-Dodecahydrat bei ca. 35°C und Lithiumnitrat-Trihydrat bei ca. 28°C, d.h. alle unterhalb der Körpertemperatur (ca. 37°C). Die Aktivierung und die Wasserfreisetzung erfolgt dabei durch Erwärmen über den Schmelzpunkt, beispielsweise etwa auf Körpertemperatur.

Die Zusammensetzungen auf Basis von niedrigschmelzenden Salzhydraten eignen sich für alle Prozesse, bei denen durch Erwärmen auf etwa Körpertemperatur eine im Wesentlichen aus Wasser bestehende flüssige Phase freigesetzt wird, die dann beispielsweise als Lösungsmittel für wasserlösliche Substanzen, oder auch zur Aktivierung wasseraktivierbarer TTS, oder zur Gaserzeugung, wie z.B. der Bildung von Kohlendioxid aus Gemischen von festen organischen Säuren und anorganischen Carbonaten dienen kann.

Bei den Zusammensetzungen, bei denen die Freisetzung der im Wesentlichen aus Wasser bestehenden flüssigen Phase durch Erwärmen induziert wird, kann die "zeitliche Verzögerung der Freisetzung" durch die Temperatur der Wärmequelle und die zugeführte Wärmemenge beliebig gesteuert werden. In gekühltem Zustand sind die Ölsäure-Wasser-Gemische, und unterhalb ihres Schmelzpunktes sind die niedrigschmelzenden Salzhydrate über eine längeren Zeitraum lagerbar, ohne dass eine Freisetzung der im Wesentlichen aus Wasser bestehenden flüssigen Phase erfolgt.

Bei der verschlossenen flüssigkeitsdichten Verpackung gemäß der vorliegenden Erfindung handelt es sich vorzugsweise um eine Folienverpackung, wie einen Beutel, vorzugsweise einen versiegelter Flachbeutel, insbesondere einen Dreirand- oder Vierrandsiegelbeutel. Der Flachbeutel entsteht durch eine einfache Faltung der Folie in Folienlaufrichtung. Je nach Bedarf kann der Flachbeutel als Dreirandsiegelbeutel oder Vierrandsiegelbeutel ausgeführt werden. Während der Dreirandsiegelbeutel nur an den Seiten und der Kopfseite gesiegelt ist, erhält der Vierrandsiegelbeutel zusätzlich eine Siegelnaht an der Unterseite. Hierdurch hat der Vierrandsiegelbeutel bei gleichen Außenmaßen zwar ein geringeres Volumen als der Dreirandsiegelbeutel, dafür ggf. über einen besseren Schutz des Beutels vor Beschädigungen.

Voraussetzung für die Eignung von Kunststoffen für Herstellung von flüssigkeitsdichten Folienverpackungen ist neben günstigen Werkstoffeigenschaften wie mechanische Festigkeit, geringes Eigengewicht und ausreichend gute Verarbeitbarkeit aus hygienischen Gründen in erster Linie die gute Sterilisierbarkeit. Diese Anforderungen werden z.B. von Polyethylen, Polypropylen, Polyvinylchlorid, Polystyrol, Polymethacrylaten, Polyamiden, Polyestern und Polycarbonaten in ausreichender Weise erfüllt. Diese Polymere sind als Beutelmaterialien geeignet. Bevorzugt sind Polyethlen (PE, Low Density Polyethylen und High Density Polyethylen) und Polypropylen (PP). OPP und BOPP sind Abwandlungen von PP um die Eigenschaften zu verändern. Hier wird unterschieden zwischen OPP (in einer Richtung vorgestrecktes PP) und BOPP (zweiseitig vorgestrecktes PP). Diese Polymere sind ebenfalls gut geeignet.

Das vor Verschließen der Verpackung der vorzugsweise formfesten Zubereitung gegebenenfalls in ausreichender Menge bereits zugesetzte Aktivierungsmittel oder eine Temperaturerhöhung induziert nach dem Verschließen der Verpackung die zeitlich verzögerte Freisetzung flüssigen Phase, die aus Wasser und ggf. darin gelösten weiteren Bestandteilen aus der vorzugsweise formfesten wasserhaltigen Zusammensetzung besteht.

Zusätzlich eingeschlossen in den Beutel ist ggf. mindestens ein mechanisches Mittel zur Öffnung, Perforation oder Zerstörung der Verpackung. Dieses mechanische Mittel kann aber auch auf der Außenseite des Beutels, z.B. durch Kleben befestigt sein. Statt des mechanischen Mittels zur Öffnung, Perforation oder Zerstörung der Verpackung oder zusätzlich kann in den Beutel auch ein anderer Öffnungs- oder Zerstörungsmechanismus, wie eine Schwächungsstelle oder eine Sollbruchstelle, integriert sein.

Die Erfindung betrifft auch ein Verfahren zur Herstellung des vorgenannten Systems, das dadurch gekennzeichnet ist, dass man die vorzugsweise formfeste wasserhaltige Zusammensetzung, gegebenenfalls eine für die zeitlich verzögerte Freisetzung der im Wesentlichen aus Wasser bestehenden flüssigen Phase ausreichende Menge eines Aktivierungsmittels und gegebenenfalls mindestens ein mechanisches Mittel zur Öffnung, Perforation oder Zerstörung der Verpackung in eine feuchtigkeitsdichte Folienverpackung gibt und diesen Folienverpackung anschließend verschließt, vorzugsweise durch feuchtigkeitsdichtes Siegeln.

Der vorliegenden Erfindung lag daher auch die Aufgabe zu Grunde, ein TTS bereitzustellen, bei dem nach bestimmungsgemäßem Gebrauch eine missbräuchliche Entnahme des restlichen Wirkstoffs möglichst vollständig unmöglich bleibt und das zusätzlich über eine längere Zeitdauer problemlos lagerfähig ist und darüber hinaus Transportschäden durch unbeabsichtigten Austritt von in Flüssigkeit gelöstem Agens nicht vorkommen lässt.

Gelöst wird diese weitere Aufgabe durch die Bereitstellung eines TTS, bevorzugt in Form eines auf die Hautoberfläche des Patienten aufzubringenden transdermalen Pflasters, das sich nach Gebrauch, d.h. nach Abnahme des TTS von der Hautoberfläche des Patienten, selbst zerstört. Sich selbst zerstörendes TTS bedeutet erfindungsgemäß, dass der in dem TTS enthaltene restliche Arzneimittelwirkstoff nach Gebrauch direkt oder indirekt zerstört, chemisch zersetzt und/oder unbrauchbar gemacht wird. Dabei ist aber auch immer gewährleistet, dass dieser Zerstörungsprozess nicht schon vor oder noch während der transdermalen Applikation des TTS gestartet wird.

Die Erfindung betrifft daher auch ein selbstzerstörendes transdermales therapeutisches System (TTS), vorzugsweise in Form eines transdermalen Pflasters, enthaltend mindestens einen Wirkstoff, mindestens ein den Wirkstoff unbrauchbar machendes wasserlösliches Agens, mindestens eine Trennung zwischen dem Wirkstoff und das den Wirkstoff unbrauchbar machenden Agens und mindestens ein mechanisches Mittel zur Öffnung, Perforation oder Zerstörung der Verpackung, das bewirkt, dass beim Abziehen des TTS nach Gebrauch die Trennung zwischen dem Wirkstoff und dem den Wirkstoff unbrauchbar machenden Agens aufgehoben wird, indem es dafür sorgt, dass aus einem oben definierten aktivierten System die im Wesentlichen aus Wasser bestehende flüssige Phase an das den Wirkstoff unbrauchbar machende Agens herantreten kann und dass so der Wirkstoff und das den Wirkstoff unbrauchbar machende Agens miteinander in Berührung kommen und durch diesen Kontakt der Wirkstoff zerstört wird, d.h. diesen vorzugsweise durch chemische Reaktion zerstört, zersetzt oder auf jeden Fall unbrauchbar macht.

Bei dem Agens kann es sich um eine Substanz oder um ein Substanzgemisch handeln, die oder das erfindungsgemäß als Feststoff oder als Paste vorliegen kann. Bevorzugt ist das Agens eine wasserlösliche Substanz, die mit dem Wirkstoff chemisch reagiert und ihn dadurch zerstört, insbesondere ein chemisches Oxidationsmittel wie z.B. anorganische Reagenzien, wie Permanganate, z.B. Kaliumpermanganat, Cer(IV)-Salze, Chromate, Nitrite, wie Kaliumnitrit, PeroxoVerbindungen, Hypohalogenide; bevorzugt Kaliumpermanganat und Kaliumnitrit. Bei gegebenem Wirkstoff wird das Agens bevorzugt auf Grund seiner chemischen Reaktionsfähigkeit mit dem Wirkstoff ausgewählt.

Bei dem Wirkstoff handelt es sich bevorzugt um einen Wirkstoff aus der Gruppe der Schmerzmittel wie z. B. Narkotika, Bevorzugt sind zu nennen Morphin, Heroin, Buprenorphin, Fentanyl, Remifentanil, Sufentanil, Alfentanyl, sowie Codein, Dihydrocodein, Desomorphin, Dextromoramid, Dextropropoxyphen, Ethylmorphin, Hydrocodon, Hydromorphon, Levomethadon, Levorphanol, Methadon, Metopon, Nalbuphin, Nicomorphin, Oxycodon, Naloxon, Oxycodon, Pentazocin, Pethidin, Tapentadol, Tilidin oder Tramadol. Bevorzugte Wirkstoffe sind Morphin, Desomorphin, Ethylmorphin, Nicomorphin, Heroin, Buprenorphin, Fentanyl, Remifentanil, Sufentanil oder Alfentanyl. Grundsätzlich können alle Opioide, die als Drogen verwendet werden, die sich durch ein hohes Abhängigkeitspotential und der lebensgefährlichen und zerstörerischen unerwünschten Wirkungen durch den Einsatz des Erfindungsgegenstands nach Anwendung unbrauchbar gemacht werden, um so einen Missbrauch verhindern.

Grundsätzlich sind auch alle anderen Kombinationen von Wirkstoff und Agens verwendbar, für die eine transdermale Applikation über ein TTS eine geeignete Darreichungsform ist.

Die Trennung zwischen dem Wirkstoff und dem Agens wird normalerweise durch eine für Flüssigkeiten durchlässige, für Feststoffe aber undurchlässige Schicht, zum Beispiel ein Papier, eine Membran oder ein Vlies bewirkt. Dabei kann das Vlies aus mineralischen Fasern, wie z.B. Glas, Mineralwolle, Basalt, tierischen Fasern wie z.B. Seide oder Wolle, pflanzlichen Fasern wie z.B. Baumwolle oder chemischen Fasern aus natürlichen (z.B. Cellulose) und/oder synthetischen Polymeren bestehen. Als synthetische Kunststoffe können hierzu Standardpolymere eingesetzt werden wie z.B. Polyamid, Polyimid, Polytetrafluorethylen, Polyethylen, Polypropylen, Polyvinylchlorid, Polyacrylate oder Polymethacrylate, Polystyrol, Polyester oder Polycarbonate.

Beim Abnehmen des Pflasters/TTS von der Haut des Patienten wird der Beutel geöffnet, perforiert oder zerstört und die Trennung zwischen Wirkstoff und Agens aufgehoben, indem der Zutritt von Wasser aus dem Beutel an das Agens stattfindet. Die Flüssigkeit tritt an das Agens heran, löst es auf und hilft so, dass das Agens durch zum Beispiel das Vlies hindurch bewegt wird, mit dem Wirkstoff in unmittelbaren Kontakt tritt und ihn dabei zerstört.

Das Mittel, das den Zutritt von Flüssigkeit bewirkt oder ermöglicht, ist ein mechanisches Mittel, das in unterschiedlichen Formen auftreten kann. Es soll damit auf jeden Fall gewährleistet sein, dass bei jeder Abnahme des TTS, und zwar unabhängig von der Abzugsrichtung, das Mittel seine bestimmungsgemäße Funktion erfüllt, nämlich direkt oder indirekt die Aufhebung der Trennung zwischen Wirkstoff und Agens zu ermöglichen, was aber zeitlich nicht schon vorher passieren darf. Dazu besitzt das Mittel eine Vielzahl von scharfen oder spitzen Bereichen.

Einfachste Ausführungsform eines solchen Mittels ist ein Stern.

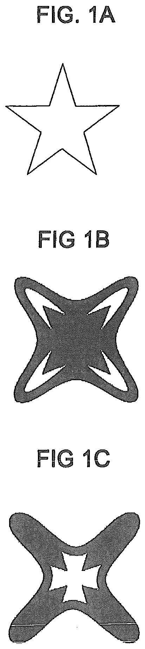

Ein Stern ist beispielhaft in Figur 1A dargestellt. Ein solcher Stern kann scharfe Spitzen, Dornen oder Kanten aufweisen, die ab einem bestimmten Biegeradius oder ab einer bestimmten mechanischen Beanspruchung des TTS zur Perforation mindestens einer benachbarten Schicht führen, die zum Beispiel eine Wand eines Flüssigkeitsvorrats oder eine Trennfolie sein kann, und so den Zutritt von Flüssigkeit bewirken oder zumindest ermöglichen.

In einer bevorzugten Ausführungsform besitzt das mechanische Mittel zur Perforation eine stumpfe Außenkontur und einen scharfen oder spitzen innenliegenden Bereich.

Beispiele für eine solche erfindungsgemäß bevorzugte Geometrie sind in Figur 1B und Figur 1C dargestellt. Beide Darstellungen zeigen Geometrien mit rundem, stumpfem und somit entschärftem äußerem Rand, die erfindungsgemäß bevorzugt eingesetzt werden. Damit besteht nämlich nicht länger die Gefahr, dass die Öffnung, Perforation oder Zerstörung der Verpackung unbeabsichtigt bei der Herstellung, bei der Lagerung, beim Transport oder während der bestimmungsgemäßen Handhabung vorzeitig passiert und somit der Wirkstoff schon vor seinem Gebrauch zerstört wird. Die spitzen, scharfen Bereiche liegen geschützt im Inneren der Geometrie. Bei großen Biegeradien und/oder bei geringer Krafteinwirkung auf das TTS wird somit die Perforation nicht eingeleitet. Erst beim Abziehen des TTS von der Haut ist der Biegeradius ausreichend klein oder es sind die zur Wirkung kommenden mechanischen Kräfte ausreichend groß um durch Verbiegen der Struktur um durch die Geometrie vorgegebene Drehpunkte die entsprechende Spitze im Innenbereich um einen Winkel bis maximal 90° aus der Ebene in Richtung zur benachbarten Schicht herauszudrehen. Auf Grund der durch die Steifigkeit des Materials erreichten Spannung im System wird eine Perforation mindestens einer benachbarten Schicht erreicht.

Dabei ist es besonders zweckmäßig, dass das mechanische Mittel, mit dem die Perforation mindestens einer benachbarten Schicht bewirkt wird, eine an die Flächenausdehnung des TTS angepasste Größe besitzt, vorzugsweise ist es nur geringfügig kleiner als die Innenfläche des TTS. Damit wird einerseits eine ausreichende Flexibilität des Systems gewährleistet, während andererseits die durch das Umbiegen erreichte Spannung in der Struktur für eine Perforation der benachbarten Schicht ausreicht. Daneben spielt auch das Verhältnis der Länge der Spitze zur Gesamtlänge des Mittels in Kraftrichtung eine Rolle. Je kürzer die Länge der Spitze ist, umso empfindlicher ist das System, da sich mit einer Verkürzung der Spitzenlänge deren Steifigkeit im Verhältnis zur Gesamtlänge des Mittels erhöht. Durch Krafteinwirkung wie Zug nach oben beim Abziehen des TTS wird die Spitze um ihren Drehpunkt/ihre Drehpunkte geschwenkt und dann in einem spitzen Winkel im Bereich von 20 bis 90° durch mindestens eine benachbarte Schicht gedrückt.

Als Material für das mechanische Mittel eignet sich zum Beispiel ein flexibler Kunststoff mit ausreichender Steifigkeit. Kunststoffe mit entsprechenden Eigenschaften sind zum Beispiel Standardpolymere wie Polyethylen oder Polypropylen, Polyester wie Polyethylentherephthalat, aber auch andere Polymere wie Cyclo-Olefin-Copolymere, Polyacrylate oder Polymethacrylate, Polytetrafluorethylen, PVC, Polycarbonat, Polystyrol, Perfluoralkoxy-Polymere (PFA), Perfluorethylenpropylen, etc. Die Materialstärke beeinflusst die Wirksamkeit beim bestimmungsgemäßen Einsatz.

Die Kunststoffe werden in Dicken von 100 bis 1000 µm, bevorzugt von 200 bis 700 µm, besonders bevorzugt von 250 bis 550 µm eingesetzt. Durch die bevorzugte Geometrie des Mittels, nämlich das Verhältnis der Länge der Spitze zur Gesamtlänge und durch die Anordnung der Drehpunkte wird trotz der Steifigkeit des Materials eine hohe Flexibilität und ein angenehmes Tragegefühl des TTS erreicht, ohne dass die Funktionalität der Selbstzerstörung verloren geht.

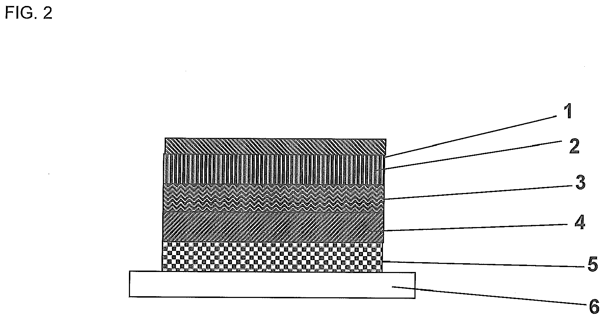

Das erfindungsgemäße selbstzerstörende TTS besitzt prinzipiell einen mehrschichtigen Aufbau, für den eine mögliche Variante in dem als Figur 2 beigefügten Ausführungsbeispiel exemplarisch erläutert wird.

Figur 2 ist eine schematische Darstellung eines senkrechten Schnitts durch ein erfindungsgemäßes TTS mit einem möglichen mehrschichtigen Aufbau. Dieser umfasst in der Darstellung mindestens eine z.B. flüssigkeitsdurchlässige obere Abdeckschicht 1 aus z.B. hautfarben eingefärbtem Gewebe, das auf seiner unteren Seite zumindest bereichsweise mit einer dünnen Kleberschicht überzogen ist, und eine untere Kleberschicht 5, die beim bestimmungsgemäßen Gebrauch des TTS in unmittelbarem Hautkontakt steht und in die der Wirkstoff eingelagert ist. Der Wirkstoff wird von dort aus in die oberste Schicht der Haut, die Epidermis, abgegeben. Nicht gezeigt wird in Figur 2 das mechanische Mittel zur Perforation.

Möglich ist auch die Ausbildung als Membranpflaster, bei dem zwischen einem Wirkstoff-Reservoir und der Haut eine klebende Membran angeordnet ist, die den Wirkstoff in die Epidermis abgibt und die in der Lage ist, die Geschwindigkeit der Abgabe zu steuern.

Unter der oberen Abdeckschicht 1 befindet sich die mindestens ein mechanische Mittel zur Perforation enthaltende verschlossenen flüssigkeitsdichten Folienverpackung 2 mit einer darin eingeschlossenen vorzugsweise formfesten wasserhaltigen Zusammensetzung, die nach Aktivierung eine im Wesentlichen aus Wasser bestehende flüssige Phase freisetzt hat. Unterhalb der Folienverpackung befindet sich ein Reservoir 3 für das Agens zur Zerstörung des Wirkstoffes, vorzugsweise ein Oxidationsmittel, wie Kaliumpermanganat in fester Form, darunter ein Vlies 4 und darunter die vorstehend schon erwähnte Kleberschicht 5 mit dem Wirkstoff. Für Lagerung und Transport des TTS ist unterhalb der Kleberschicht 5 noch eine transparente Schutzfolie 6 vorgesehen, die vor Gebrauch des TTS zu entfernen ist.

Im Übrigen können für die Herstellung des erfindungsgemäßen TTS bzw. transdermalen Pflasters alle Materialien zum Einsatz kommen, die dem Fachmann für solche Systeme bekannt sind. Für die Herstellung des erfindungsgemäßen TTS kann der Fachmann somit grundsätzlich auf die Materialien, Herstellungsverfahren und den Aufbau der aus dem Stand der Technik bekannten TTS bzw. transdermalen Pflaster zurückgreifen, die erfindungsgemäß zusätzlich eine geeignete Kombination aus Mittel und Agens aufweisen (vgl. dazu: Transdermale Pflaster; Spektrum der Wissenschaft 10/2003, 42; Transdermal Controlled Systemic Madications, Y.W. Chien, Drugs and the Pharmaceutical Sciences, Vol. 31; Polymers in Transdermal Drug Delivery Systems, S. Kandavilli et. al., Pharmaceutical Technology, May 2002, 62 - 80).

Die Erfindung wird durch die folgenden Beispiele näher erläutert ohne darauf beschränkt zu sein. Gleichwohl können spezielle, in den Beispielen beschriebene Ausgestaltungen der erfindungsgemäßen Systeme einzeln oder in Kombination miteinander als bevorzugte Merkmale für die Erfindung als solche verallgemeinert werden. Wenn nicht anders angegeben, handelt es sich bei %-Angaben um Gewichts-%.

### Herstellung von Pektin-Wafern (siehe Tabelle 1, Beispiele Nr. 1 - 4)

Destilliertes Wasser wurde im Weithalsglas im Wasserbad bei ca. 73°C mit einem Dispergierrührer bei 200 Umdrehungen pro Minute (UpM) gerührt. Pektin classic CU 701 wurde unter Rühren bei 500 UpM langsam in kleinen Portionen zugegeben. Anschließend wurde die Rührgeschwindigkeit auf 1500 UpM erhöht. Es entstand eine hellbraune, trübe Masse mit einer gestiegenen Viskosität. Calciumchlorid wurde eingewogen und unter Rühren bei einer Wasserbadtemperatur von ca. 75°C zugegeben. Anschließend wurde die Geschwindigkeit auf 2000 UpM erhöht. Nach mindestens 30 Minuten wurde die Masse mit einem Glasstab geprüft, es bildete sich spontan ein Gel. Anschließend wurde die Masse auf die silikonisierte Seite einer 100 µm Polyesterfolie aufgetragen, welche idealerweise zuvor auf eine Temperatur von 50 - 70°C aufgeheizt wurde. Die Masse härtete schnell aus und konnte mit einer zweiten Polyesterfolie mit der silikonisierten Seite abgedeckt werden. Aus diesem Laminat konnten nun Wafer hergestellt werden.

### Herstellung eines ^{®}Meyprogat-Wafers (siehe Tabelle 1, Beispiel Nr. 5)

Destilliertes Wasser wurde im Weithalsglas mit einem Flügelrührer bei 200 UpM gerührt. ^{®}Meyprogat 90 wurde unter Rühren bei 200 - 500 UpM langsam in kleinen Portionen zugegeben. Die Rührgeschwindigkeit wurde auf 2000 UpM erhöht. Die Masse wird homogen und die Viskosität steigt, bis sich die Masse nicht mehr rühren lässt. Aus der festen Masse konnten nun Wafer hergestellt werden.

### Herstellung eines Ölsäure-Wafers (siehe Tabelle 1, Beispiel Nr. 6)

Ölsäure wurde im Weithalsglas klar vorgelegt. Destilliertes Wasser wurde zugegeben und mit einem Dispergierrührer bei 1000 UpM homogenisiert. Es entstand eine milchige Emulsion. Die Emulsion wurde im Eisbad bei ca. 0,3°C weitergerührt. Nach etwa 30 Minuten entstand eine weiße, steife Masse. Anschließend wurde die Masse im Kühlschrank bei ca. 6°C gelagert. Aus der steifen Masse konnten nun Wafer hergestellt werden.

### Herstellung eines Natriumpolyacrylat-Wafers (siehe Tabelle 1, Beispiel Nr. 7)

^{®}Aronvis wurde im Weithalsglas vorgelegt. Destilliertes Wasser wurde zugegeben und mit einem Glasstab gerührt. Es entstand ein festes Gel. Aus der festen Masse konnten nun Wafer hergestellt werden.

**Tabelle 1**

| Herstellung der formfesten wasserhaltigen Zusammensetzungen | | | | | | | |
|---|---|---|---|---|---|---|---|
| Bsp. Nr. | Grundlage | Lösemittel | Salz für Gel | Kationen-Konz. | Salz für Wasserfreisetzung | Kationen-Konz. für Wasserfreisetzung | Bemerkung |
| 1 | Pektin Classic CU 701 5% | Wasser, dest. 94,7% | CaCl₂ 0,3% | 0,11% | CaCl₂ >0,3% | >0,11 % | 1), 2) |
| 2 | Pektin Classic CU 701 5% | Wasser, dest. 93,5% | Calcium-L-Lactat 1,5% | 0,28% | CaCl₂ >0% | >0% | |
| 3 | Pektin Classic CU 701 5% | Wasser, dest. 94,1% | Calcium-Citrat 0,9% | 0,20% | CaCl₂ >0% | >0% | |
| 4 | Pektin Classic CU 701 5% | Wasser, dest. 94,8% | AlCl₃ 0,2% | 0,04% | AlCl₃ >0,2% | >0,04% | |
| 5 | ^{®}Meyprogat 90 5% | Wasser, dest. 95% | --- | --- | Citro-nensäure >0% | --- | 3) |
| 6 | Ölsäure 50% | Wasser, dest. 50% | --- | --- | --- | --- | 4) |
| 7 | Natriumpolyacrylat (^{®}Avonis-Typen) 18-26% | Wasser, dest. 74-82% | --- | --- | Citro-nensäure >0% | --- | 5) |

Bei Pektin Classic CU 701 handelt es sich um ein NV-Pektin (niederverestertes Pektin).
1) Die Gele setzen Wasser frei, wenn man das entsprechende Salz hinzugibt.
2) Wasserfreisetzung nach Zugabe von CaCl₂ zwischen 40 - 60%, zeitlich unabhängig (gilt für Kationen-Konz. von 7,8 - 11,1%, in Abhängigkeit von der Verteilung der Kationen auf dem Gel)
3) Es bildet sich ein nicht festes Gel, Wasser wird nach Zugabe von Citronensäure freigesetzt.
4) Eine Wasser-Ölsäure Emulsion heruntergekühlt während des Rührens, setzt bei Raumtemperatur Wasser frei
5)Es bildet sich ein festes Gel, Wasser wird nach Zugabe von Citronensäure freigesetzt.

### Herstellung der Synärese-Pektin-Wafer der Beispiele Nr. 8 - 17 von Tabelle 2

Diese Versuche wurden in PE-Beuteln mit Zipp-Verschluss durchgeführt. Dazu wurden Quadrate der Größe von ca. 10 cm² aus dem Pektin-Wafer-Laminat (siehe Tabelle 1, Beispiel Nr. 1 -4) geschnitten. Von den nun entstandenen Wafern wurde die zweite Polyesterfolie entfernt und weiteres Calciumchlorid aufgestreut. Anschließend wurden die bestreuten Wafer einzeln zur weiteren Beobachtung in die PE-Beutel eingefügt und die Zipp-Verschlüsse verschlossen.

**Tabelle 2**

| Versuchsreihe zur Bestimmung des Wasserverlustes eines Pektin-Wafers durch Synärese, hervorgerufen durch die Zugabe von CaCl₂ (eingesetztes Pektin: Pektin Classic CU 701 5%) | | | | | | |
|---|---|---|---|---|---|---|
| Bsp. Nr. | PE-Beutel Tara [g] | Gel+ Beutel [g] | Gel-Muster 10 cm² [g] | Einwaage CaCl₂ [g] | Zeitpunkt [h] | Wasser [g] |
| 8 | 1,7122 | 2,9812 | 1,2690 | 0,3476 | 2 | 0,5060 |
| 9 | 1,6966 | 2,8983 | 1,2017 | 0,3684 | 22 | 0,5347 |
| 10 | 1,7123 | 2,9218 | 1,2095 | 0,4000 | 46 | 0,6300 |
| 11 | 1,7363 | 2,9724 | 1,2361 | 0,4045 | 70 | 0,5300 |
| 12 | 1,7446 | 2,8032 | 1,0585 | 0,2864 | 144 | 0,6379 |
| 13 | 1,7364 | 3,0426 | 1,3062 | 0,4263 | 168 | 0,6079 |
| 14 | 1,7645 | 3,0407 | 1,2762 | 0,5581 | 192 | 0,7452 |
| 15 | 1,7423 | 3,0303 | 1,2880 | 0,5040 | 216 | 0,7765 |
| 16 | 1,7437 | 2,9705 | 1,2268 | 0,3980 | 240 | 0,5000 |
| 17 | 1,7249 | 2,9757 | 1,2508 | 0,3774 | 312 | 0,3786 |

### Beispiel 18

### Herstellung eines selbstzerstörenden TTS

Zu 1,14 kg einer Lösung eines selbst-vernetzenden Polyacrylates, bestehend aus den Monomeren 2-Ethylhexylacrylat, Vinylacetat, Butylacrylat und Acrylsäure in der Mischung der organischen Lösemittel Ethylacetat, Heptan und Isopropanol/Toluol, wurden 100 g Lävulinsäure, 150 g Oleyloleat, 100 g Polyvinylpyrrolidon, 150 g Ethanol, 200 g Ethylacetat und 100 g Buprenorphin Base zugegeben. Diese Mischung wurde bis zur Homogenisierung über eine Zeitdauer von etwa 2 Stunden gerührt. Nach dem Homogenisieren wurde die Mischung auf die silikonisierte Seite einer 100 µm Polyesterfolie aufgetragen, anschließend daran wurden die Lösemittel durch 10-minütiges Trocknen bei 70°C im Trockenschrank entfernt. Die Strichstärke in der Beschichtung wurde so gewählt, dass nach Entfernen der Lösemittel ein Flächengewicht von ca. 80 g/m² resultierte. Nach dem Entfernen der Lösemittel wurde das Laminat bestehend aus silikonisierter Polyesterfolie und wirkstoffhaltiger Polymerschicht mit einer zweiten, schwächer silikonisierten Polyesterfolie abgedeckt. Danach wurde das so entstandene Laminat in Quadrate der Kantenlänge 5 x 5 cm zugeschnitten. Die 5 x 5 cm große silikonisierte Polyesterfolie wurde dann auf der einen Seite des Laminates entfernt und es wurde mittig ein zum Beispiel 4 x 4 cm großes saugfähiges, flüssigkeitsdurchlässiges Material, z.B. ein Vlies, aufgeklebt. Auf das saugfähige, flüssigkeitsdurchlässige Vlies wurde dann ein randseitig geprägter Beutel aus Filterpapier, gefüllt mit Kaliumpermanganat in Pulverform, aufgelegt, der so gestaltet war, dass er in seiner Gesamtfläche kleiner war als die wirkstoffhaltige Polymerschicht.

Ohne die Erfindung einzuschränken, kann der Beutel Maße von 4 x 4 cm aufweisen. Nun wurde ein rundum versiegelter, flüssigkeitsdichter Beutel in der Größe 5 x 5 cm versehen mit einem vierzackigen inversen Malteserstern in der Art, wie er in Figur 1C dargestellt ist, aus hartem Polymermaterial und einer Menge von 1,5 ml Wasser (freigesetzt durch Synärese aus dem Gel-Wafer des Beispiels Nr.1) eingeklebt. Beim Abziehen des gebrauchten TTS von der Haut des Patienten bohrt sich mindestens eine Spitze des Maltesers durch die untere Wand des Flüssigkeitsbeutels und bewirkt so den Austritt des Wassers, das mit dem darunter angeordneten Kaliumpermanganat sofort in Kontakt tritt.

Wird das TTS im Rahmen seines bestimmungsgemäßen Verwendungszweckes appliziert, muss zunächst einmal die silikonisierte Polyesterschicht (Schutzfolie) entfernt werden, was leicht möglich ist. Wird das TTS auf die Haut eines Patienten aufgeklebt, dann bleibt der mit Wasser gefüllte Beutel zunächst unbeschädigt. Ein Zutritt von Flüssigkeit zum Kaliumpermanganatpulver ist nicht möglich. Wird aber nach der Applikationsdauer von 1 bis 7 Tagen das TTS von der Haut des Patienten abgezogen, dann bohrt sich mindestens eine Spitze des vierzackigen inversen Maltesersterns auf Grund der Steifigkeit des Polymermaterials durch die Folie des Beutels und perforiert diese zwangsläufig. Durch die Geometrie des Maltesers ist sichergestellt, dass der Beutel in jedem Falle perforiert wird, egal in welcher Richtung das TTS vom Patienten entfernt wird.

Das Wasser kann nun durch die Abdeckfolie und die Perforation in der Trennschicht in das TTS eindringen, das

Kaliumpermanganat auflösen und durch das saugfähige Vlies hindurch innerhalb kurzer Zeit zu dem restlichen Wirkstoff in der unteren Kleberschicht hin transportieren. Dort wird sofort ein Oxydationsprozess gestartet, der im Falle von z.B. Buprenorphin zu dessen oxidativer Zerstörung führt. Dadurch wird sichergestellt, dass der Wirkstoff nicht missbraucht werden kann.

## Patentansprüche

1. System bestehend aus einer verschlossenen flüssigkeitsdichten Verpackung und einer darin eingeschlossenen vorzugsweise formfesten wasserhaltigen Zusammensetzung, die gegebenenfalls eine ausreichende Menge eines Aktivierungsmittels enthält, wobei die Zusammensetzung mit zeitlicher Verzögerung eine im Wesentlichen aus Wasser bestehende flüssige Phase freisetzt,
worin es sich bei der vorzugsweise formfesten wasserhaltigen Zusammensetzung um ein Hydrogel auf Basis eines niederveresterten Pektins oder eines niederveresterten, amidierten Pektins handelt, und wobei die Zusammensetzung eine für die zeitlich verzögerte Freisetzung der im Wesentlichen aus Wasser bestehenden flüssigen Phase ausreichende Menge eines Erdalkali- oder Erdmetallsalzes, vorzugsweise eines Calciumsalzes, insbesondere an Calciumchlorid als Aktivierungsmittel enthält,
oder worin es sich bei der vorzugsweise formfesten wasserhaltigen Zusammensetzung um ein Hydrogel auf Basis eines depolymerisierten Guargummiprodukts oder eines Polyacrylats handelt, und wobei die Zusammensetzung eine für die zeitlich verzögerte Freisetzung der im Wesentlichen aus Wasser bestehenden flüssigen Phase ausreichende Menge einer organischen Säure, vorzugsweise an Citronensäure als Aktivierungsmittel enthält,
oder worin es sich bei der vorzugsweise formfesten wasserhaltigen Zusammensetzung um ein gekühltes Ölsäure-Wasser-Gemisch oder ein niedrigschmelzendes Salzhydrat, vorzugsweise Calciumchlorid-Hexahydrat, Mangansulfat-Tetrahydrat, Natriumhydrogenphosphat-Dodecahydrat oder Lithiumnitrat-Trihydrat handelt, wobei die zeitlich verzögerte Freisetzung der im Wesentlichen aus Wasser bestehenden flüssigen Phase beim Erwärmen der Zusammensetzung erfolgt.

2. System gemäß Anspruch 1, worin die verschlossene flüssigkeitsdichte Verpackung eine Folienverpackung ist.

3. System gemäß Anspruch 1 oder 2, worin die verschlossene flüssigkeitsdichte Verpackung ein Beutel, vorzugsweise ein versiegelter Flachbeutel, insbesondere ein Dreirand- oder ein Vierrandsiegelbeutel ist.

4. System gemäß einem der Ansprüche 1 bis 3, in dem zusätzlich mindestens ein mechanisches Mittel zur Öffnung, Perforation oder Zerstörung der Verpackung eingeschlossen ist.

5. Verfahren zur Herstellung eines Systems gemäß einem der Ansprüche 1 bis 4, **dadurch gekennzeichnet, dass** man die formfeste wasserhaltige Zusammensetzung, gegebenenfalls ein Aktivierungsmittel und gegebenenfalls mindestens ein mechanisches Mittel zur Öffnung, Perforation oder Zerstörung der Verpackung in eine feuchtigkeitsdichte Verpackung, vorzugsweise Folienverpackung gibt und diese Verpackung anschließend verschließt, vorzugsweise durch feuchtigkeitsdichtes Siegeln.

6. Selbstzerstörendes transdermales therapeutisches System (TTS) enthaltend mindestens einen Wirkstoff, mindestens ein den Wirkstoff unbrauchbar machendes wasserlösliches Agens, mindestens eine Trennung zwischen dem Wirkstoff und das den Wirkstoff unbrauchbar machenden Agens und mindestens ein mechanisches Mittel zur Öffnung, Perforation oder Zerstörung der Verpackung, das bewirkt, dass beim Abziehen des TTS nach Gebrauch die Trennung zwischen dem Wirkstoff und dem den Wirkstoff unbrauchbar machenden Agens aufgehoben wird, indem es dafür sorgt, dass aus einem aktivierten System gemäß einem der Ansprüche 1 bis 4 die im Wesentlichen aus Wasser bestehende flüssige Phase an das den Wirkstoff unbrauchbar machende Agens herantreten kann und dass so der Wirkstoff und das den Wirkstoff unbrauchbar machende Agens miteinander in Berührung kommen und durch diesen Kontakt der Wirkstoff zerstört wird.

7. Selbstzerstörendes transdermales therapeutisches System nach Anspruch 6 , **dadurch gekennzeichnet, dass** es ein transdermales Pflaster ist.

8. Selbstzerstörendens transdermales therapeutisches System nach Anspruch 6 oder 7, **dadurch gekennzeichnet, dass** es das den Wirkstoff unbrauchbar machende Agens in fester oder in pastöser Form enthält.

9. Selbstzerstörendes transdermales therapeutisches System nach einem der Ansprüche 4 bis 6, **dadurch gekennzeichnet, dass** der Wirkstoff und das den Wirkstoff unbrauchbar machende Agens nach dem Abziehen des TTS von der Haut des Patienten in Anwesenheit einer mobilen Phase chemisch miteinander reagieren.

10. Selbstzerstörendens transdermales therapeutisches System nach einem der Ansprüche 6 bis 9, **dadurch gekennzeichnet, dass** der Wirkstoff ein Schmerzmittel oder Narkotikum, vorzugsweise Morphin, Desomorphin, Ethylmorphin, Nicomorphin, Heroin, Buprenorphin, Fentanyl, Remifentanil, Sufentanil oder Alfentanyl ist.

11. Selbstzerstörendes transdermales therapeutisches System nach einem der Ansprüche 4 bis 8, **dadurch gekennzeichnet, dass** das den Wirkstoff unbrauchbar machende Agens ein wasserlösliches Oxidationsmittel, vorzugsweise Kaliumpermanganat ist.

12. Selbstzerstörendes transdermales therapeutisches System nach einem der Ansprüche 6 bis 11, **dadurch gekennzeichnet, dass** das mechanische Mittel zur Öffnung, Perforation oder Zerstörung der Verpackung
- eine Vielzahl von scharfen oder spitzen Bereichen besitzt, oder
- eine sternförmige Form besitzt, oder
- eine stumpfe Außenkontur und einen scharfen oder spitzen innenliegenden Bereich besitzt.

13. Selbstzerstörendes transdermales therapeutisches System nach Anspruch 12, **dadurch gekennzeichnet, dass** bei dem mechanische Mittel zur Öffnung, Perforation oder Zerstörung der Verpackung beim Abziehen des TTS von der Haut durch Verbiegen seiner Struktur um durch ihre Geometrie vorgegebene Drehpunkte mindestens eine entsprechend angeordnete Spitze im Innenbereich um einen Winkel bis maximal 90° aus der Ebene in Richtung zur benachbarten Schicht herausgedreht wird und so eine Perforation mindestens einer benachbarten Schicht bewirkt.

14. Verwendung eines selbstzerstörenden transdermalen therapeutischen Systems nach einem oder nach mehreren der Ansprüche 6 bis 13 in der Schmerztherapie.

15. Verwendung eines Systems gemäß einem der Ansprüche 1 bis 4 bei der Herstellung eines selbstzerstörenden transdermalen therapeutischen Systems.

## Claims

1. System consisting of a closed, liquid-tight packaging and of a preferably dimensionally stable hydrous composition enclosed therein and optionally comprising a sufficient amount of an activating agent, where the composition releases, with a temporal delay, a liquid phase consisting substantially of water,
wherein the preferably dimensionally stable hydrous composition is a hydrogel based on a low-ester pectin or on a low-ester amidated pectin, and where the composition comprises as activating agent an alkaline earth or earth metal salt, preferably a calcium salt, more particularly calcium chloride, in an amount sufficient for the temporally delayed release of the liquid phase consisting substantially of water,
or wherein the preferably dimensionally stable hydrous composition is a hydrogel based on a depolymerized guar gum product or on a polyacrylate, and where the composition comprises as activating agent an organic acid, preferably citric acid, in an amount sufficient for the temporally delayed release of the liquid phase consisting substantially of water,
or wherein the preferably dimensionally stable hydrous composition is a cooled oleic acid/water mixture or a low-melting salt hydrate, preferably calcium chloride hexahydrate, manganese sulfate tetrahydrate, sodium hydrogenphosphate dodecahydrate or lithium nitrate trihydrate, where the temporally delayed release of the liquid phase consisting substantially of water takes place when the composition is heated.

2. System according to Claim 1, wherein the closed liquid-tight packaging is a film packaging.

3. System according to Claim 1 or 2, wherein the closed liquid-tight packaging is a pouch, preferably a sealed flat pouch, more particularly a three-edge or a four-edge sealed pouch.

4. System according to any of Claims 1 to 3, further including at least one mechanical means for opening, perforating or destroying the packaging.

5. Method for producing a system according to any of Claims 1 to 4, **characterized in that** the dimensionally stable hydrous composition, optionally an activating agent, and optionally at least one mechanical means for opening, perforating or destroying the packaging are placed into a moisture-tight packaging, preferably film packaging, and this packaging is subsequently closed, preferably by moisture-tight sealing.

6. Self-destructing transdermal therapeutic system (TTS) comprising at least one active ingredient, at least one water-soluble agent that renders the active ingredient unusable, at least one separation between the active ingredient and the agent that renders the active ingredient unusable, and at least one mechanical means for opening, perforating or destroying the packaging, having the effect that when the TTS is removed after use, the separation between the active ingredient and the agent that renders the active ingredient unusable is undone, by ensuring that from an activated system according to any of Claims 1 to 4, the liquid phase consisting substantially of water is able to reach the agent that renders the active ingredient unusable and that in this way the active ingredient and the agent that renders the active ingredient unusable come into contact with one another and this contact destroys the active ingredient.

7. Self-destructing transdermal therapeutic system according to Claim 6, **characterized in that** it is a transdermal patch.

8. Self-destructing transdermal therapeutic system according to Claim 6 or 7, **characterized in that** it comprises the agent that renders the active ingredient unusable in solid or in pasty form.

9. Self-destructing transdermal therapeutic system according to any of Claims 4 to 6, **characterized in that** after the removal of the TTS from the skin of the patient, the active ingredient and the agent that renders the active ingredient unusable react chemically with one another in the presence of a mobile phase.

10. Self-destructing transdermal therapeutic system according to any of Claims 6 to 9, **characterized in that** the active ingredient is an analgesic or narcotic, preferably morphine, desomorphine, ethylmorphine, nicomorphine, heroin, buprenorphine, fentanyl, remifentanil, sufentanil or alfentanyl.

11. Self-destructing transdermal therapeutic system according to any of Claims 4 to 8, **characterized in that** the agent that renders the active ingredient unusable is a water-soluble oxidizing agent, preferably potassium permanganate.

12. Self-destructing transdermal therapeutic system according to any of Claims 6 to 11, **characterized in that** the mechanical means for opening, perforating or destroying the packaging
- possesses a multiplicity of sharp or pointed regions, or
- possesses a stellate shape, or
- possesses a blunt outer contour and a sharp or pointed inlying region.

13. Self-destructing transdermal therapeutic system according to Claim 12, **characterized in that**, with regard to the mechanical means for opening, perforating or destroying the packaging, on removal of the TTS from the skin, by distortive bending of its structure at least one correspondingly arranged point in the inner region is rotated, around pivot points dictated by its geometry, by an angle of up to a maximum of 90° out of the plane in the direction of the adjacent layer, and in this way brings about perforation of at least one adjacent layer.

14. Use of a self-destructing transdermal therapeutic system according to one or according to two or more of Claims 6 to 13 in pain therapy.

15. Use of a system according to any of Claims 1 to 4 in the production of a self-destructing transdermal therapeutic system.

## Revendications

1. Système constitué d'un emballage fermé étanche aux liquides et d'une composition aqueuse de préférence dimensionnellement stable enfermée dans celui-ci, qui contient éventuellement une quantité suffisante d'un agent activeur, la composition libérant une phase liquide constituée essentiellement d'eau avec un retard dans le temps,
dans lequel la composition aqueuse de préférence dimensionnellement stable est un hydrogel à base d'une pectine faiblement estérifiée ou d'une pectine faiblement estérifiée et amidée, et dans lequel la composition contient une quantité suffisante d'un sel de métal alcalino-terreux ou terreux pour la libération retardée dans le temps de la phase liquide constituée essentiellement d'eau, de préférence un sel de calcium, notamment du chlorure de calcium, comme agent activeur,
ou dans lequel la composition aqueuse de préférence dimensionnellement stable est un hydrogel à base d'un produit de gomme de guar dépolymérisé ou d'un polyacrylate, et dans lequel la composition contient une quantité suffisante d'un acide organique pour la libération retardée dans le temps de la phase liquide constituée essentiellement d'eau, de préférence de l'acide citrique, comme agent activeur,
ou dans lequel la composition aqueuse de préférence dimensionnellement stable est un mélange réfrigéré d'acide oléique et d'eau ou un sel hydraté à bas point de fusion, de préférence du chlorure de calcium hexahydraté, du sulfate de manganèse tétrahydraté, de l'hydrogénophosphate de sodium dodécahydraté ou du nitrate de lithium trihydraté, dans lequel la libération retardée dans le temps de la phase liquide constituée essentiellement d'eau se produit lors du chauffage de la composition.

2. Système selon la revendication 1, dans lequel l'emballage fermé étanche aux liquides est un emballage en feuille.

3. Système selon la revendication 1 ou 2, dans lequel l'emballage fermé étanche aux liquides est un sac, de préférence un sac plat scellé, en particulier un sac scellé à trois ou quatre côtés.

4. Système selon l'une quelconque des revendications 1 à 3, dans lequel au moins un moyen mécanique pour ouvrir, perforer ou détruire l'emballage est en outre inclus.

5. Procédé de fabrication d'un système selon l'une quelconque des revendications 1 à 4, **caractérisé en ce que** l'on place la composition aqueuse dimensionnellement stable, éventuellement un agent activeur et éventuellement au moins un moyen mécanique pour ouvrir, perforer ou détruire l'emballage, dans un emballage étanche à l'humidité, de préférence un emballage en feuille, et **en ce que** cet emballage est ensuite fermé, de préférence par un scellage étanche à l'humidité.

6. Système thérapeutique transdermique autodestructeur (STT) comprenant au moins un principe actif, au moins un agent hydrosoluble rendant le principe actif inutile, au moins une barrière entre le principe actif et l'agent rendant le principe actif inutile, et au moins un moyen mécanique pour ouvrir, perforer ou détruire l'emballage, qui fait que, lors du retrait du STT après utilisation, la barrière entre le principe actif et l'agent rendant le principe actif inutile est supprimée, en s'assurant que la phase liquide constituée essentiellement d'eau sortant d'un système activé selon l'une quelconque des revendications 1 à 4 peut s'approcher de l'agent rendant le principe actif inutile, et que de cette façon, le principe actif et l'agent rendant le principe actif inutile entrent en contact l'un avec l'autre et le principe actif est détruit à la suite de ce contact.

7. Système thérapeutique transdermique autodestructeur selon la revendication 6, **caractérisé en ce qu'**il s'agit d'un patch transdermique.

8. Système thérapeutique transdermique autodestructeur selon la revendication 6 ou 7, **caractérisé en ce qu'**il contient l'agent rendant le principe actif inutile sous forme solide ou pâteuse.

9. Système thérapeutique transdermique autodestructeur selon l'une des revendications 4 à 6, **caractérisé en ce que** le principe actif et l'agent rendant le principe actif inutile réagissent chimiquement entre eux en présence d'une phase mobile après que le STT a été retiré de la peau du patient.

10. Système thérapeutique transdermique autodestructeur se-Ion l'une des revendications 6 à 9, **caractérisé en ce que** le principe actif est un antalgique ou narcotique, de préférence la morphine, la désomorphine, l'éthylmorphine, la nicomorphine, l'héroïne, la buprénorphine, le fentanyl, le rémifentanil, le sufentanil ou l'alfentanyl.

11. Système thérapeutique transdermique autodestructeur selon l'une des revendications 4 à 8, **caractérisé en ce que** l'agent rendant le principe actif inutile est un agent oxydant hydrosoluble, de préférence le permanganate de potassium.

12. Système thérapeutique transdermique autodestructeur selon l'une des revendications 6 à 11, **caractérisé en ce que** le moyen mécanique pour ouvrir, perforer ou détruire l'emballage
- présente une multitude de zones aiguës ou pointues, ou
- a une forme d'étoile, ou
- a un contour extérieur émoussé et une zone intérieure aiguë ou pointue.

13. Système thérapeutique transdermique autodestructeur selon la revendication 12, **caractérisé en ce que** lorsque le STT est arraché de la peau, en pliant la structure du moyen mécanique pour ouvrir, perforer ou détruire l'emballage autour de points d'articulation prédéterminés par sa géométrie, au moins une pointe de celle-ci disposée de manière correspondante dans la zone intérieure est tourné hors du plan autour d'un angle allant jusqu'à 90° en direction de la couche adjacente, provoquant ainsi une perforation d'au moins une couche adjacente.

14. Utilisation d'un système thérapeutique transdermique autodestructeur selon une ou plusieurs des revendications 6 à 13 dans le traitement de la douleur.

15. Utilisation d'un système selon l'une quelconque des revendications 1 à 4 pour la fabrication d'un système thérapeutique transdermique autodestructeur.
